# EUROPEAN PATENT SPECIFICATION

(11) **EP 4 532 003 B1**
(45) Date of publication and mention of the grant of the patent: **17.09.2025**
(21) Application number: 23726519.4
(22) Date of filing: 17.05.2023
(51) Int. Cl.: A61N 5/06, B26B 19/14, B26B 19/46, A61B 18/00

(54) **ARRANGEMENT OF A LIGHT SOURCE AND A CARRIER THEREOF IN A HOUSING OF A SKIN TREATMENT DEVICE**
ANORDNUNG EINER LICHTQUELLE UND EINES TRÄGERS DAVON IN EINEM GEHÄUSE EINER HAUTBEHANDLUNGSVORRICHTUNG
AGENCEMENT D'UNE SOURCE LUMINEUSE ET DE SON SUPPORT DANS UN BOÎTIER D'UN DISPOSITIF DE TRAITEMENT DE LA PEAU

(30) Priority: 25.05.2022 EP 22175321
(43) Date of publication of application: 09.04.2025
(73) Proprietor: Koninklijke Philips N.V., 5656 AG Eindhoven (NL)
(72) Inventor: BOAMFA, Marius Iosif, 5656 AG Eindhoven (NL); VARGHESE, Babu, 5656 AG Eindhoven (NL); PALERO, Jonathan Alambra, 5656 AG Eindhoven (NL); ZJIROECHA, Nikolaj Vasiljevitsj, 5656 AG Eindhoven (NL)
(74) Representative: Philips Intellectual Property & Standards
(86) International application number: PCT/EP2023/063222
(87) International publication number: WO 2023/227434

(56) References cited:
- WO-A1-2022/069324
- US-A1- 2008 119 913
- US-B2- 8 876 809

## Description

### FIELD OF THE INVENTION

The invention relates to a skin treatment device comprising a housing provided with a skin-contacting surface arranged to be in contact with skin of a user during use of the device, and at least one light source arranged in the housing for generating treatment light to be applied to the skin via a light-transmitting portion of the skin-contacting surface which is optically transparent.

Further, the invention relates to a shaving unit of an electric shaver, comprising a supporting member, at least one hair-cutting unit supported by the supporting member, and at least one skin treatment device as mentioned here before. The invention also relates to an electric shaver comprising a main body and such a shaving unit, coupled to the main body.

### BACKGROUND OF THE INVENTION

A skin treatment device as mentioned here before is known in the art. For example, WO 2022/069324 A1 discloses a skin-heating unit for use in a shaving unit of an electric shaver. The shaving unit further comprises at least one hair-cutting unit and a base member supporting the at least one hair-cutting unit. The hair-cutting unit is of the type comprising an external cutting member which has hair-entry openings in a cutting track surface and an internal cutting member which has at least one hair-cutting element and which is movable relative to the external cutting member. The skin-heating unit comprises a skin-contacting member having a skin-contacting surface arranged adjacent to the at least one hair-cutting unit to contact the skin during use of the shaving unit, and at least one infrared or near-infrared light source integrated in the skin-contacting member, which light source is arranged to emit infrared or near-infrared light via the skin-contacting surface which is optically transparent. The light source is mounted on a printed circuit board (PCB), and the assembly of the light source and the PCB is embedded in an optically transparent and thermally conductive potting material with the light source mounted on a side of the PCB facing the skin-contacting surface, so that the light generated by the light source directly reaches the skin-contacting surface via the potting material.

A reason for equipping a shaving unit with a skin-heating unit such as known from WO 2022/069324 A1 is found in the fact that exposure of hairs to infrared or near-infrared light helps to soften the hairs. In general, exposure of hairs to infrared or near-infrared light during a shaving action improves the comfort experienced by the user. Also, infrared or near-infrared light may have a beneficial effect on the skin, stimulating the skin and invoking a radiant look of the skin. The fact is that the skin-heating unit is functional to achieve an effect referred to as deep optical-thermal heating. Deep optical-thermal heating is different from conductive thermal heating by a heat applicator including hot elements in conductive thermal contact with the skin, because the penetration depth of light into the skin is relatively high. The skin is heated over the entire depth of light penetration without needing to rely on thermal conductivity of both the heat applicator and the skin. In the skin-heating unit known from WO 2022/069324 A1, the light source is thermally coupled to the skin-contacting surface via said optically transparent and thermally conductive potting material, so that, in addition to the deep optical-thermal heating of the skin obtained by the infrared or near-infrared light during operation, the skin is also heated by thermal conduction to the skin of residual heat generated by the light source. In this respect, it is noted that in general, any light source has a limited efficiency in converting electric power into optical power, and part of the electric power supplied to a light source is converted into thermal energy, so that a light source generates not only light but also residual heat during operation.

Two major issues in the field of heating skin via both optical-thermal heating and thermal conductive heating by means of a device such as disclosed by WO 2022/069324 A1, configured to contact the skin at the position of an optically transparent skin-contacting surface and to emit light such as infrared or near-infrared light via the skin-contacting surface during operation, are insufficient optical spreading of the light over the skin-contacting surface and loss of the residual heat generated by the at least one light source of the device. As a result of insufficient optical spreading of the light over the skin-contacting surface, optical hot spots are created, which may cause the user to experience an uncomfortable pain sensation. Loss of the residual heat generated by the light source is related to the fact that the light source is located at a distance from the skin-contacting surface, and that part of the generated heat is absorbed by the material that is present between the light source and the skin-contacting surface or conducted to other parts of the device. In view thereof, it is an object of the invention to provide a skin treatment device which is designed such that the creation of optical hot spots during operation is limited or even avoided and that the residual heat generated by the light source is efficiently conducted to the skin-contacting surface to heat the skin under treatment.

### SUMMARY OF THE INVENTION

The invention provides a skin treatment device comprising:
- a housing provided with a skin-contacting surface arranged to be in contact with skin of a user during use of the device;
- at least one light source arranged in the housing for generating treatment light to be applied to the skin via a light-transmitting portion of the skin-contacting surface which is optically transparent;
- a carrier having a first side on which the light source is mounted, said first side facing away from the skin-contacting surface;
- an optical reflector accommodated by the housing and arranged for reflecting the treatment light generated by the at least one light source towards the light-transmitting portion of the skin-contacting surface; wherein:
- at least a portion of a second side of the carrier opposite to the first side of the carrier:
   i) is mounted to an inner side of a wall portion of the housing, said wall portion including part of the skin-contacting surface, wherein said part of the skin-contacting surface is located on an outer side of said wall portion which is opposite to said inner side of said wall portion; or
   ii) constitutes part of the skin-contacting surface.

The invention is about the arrangement of a light source and a carrier thereof in a housing of a skin treatment device. It follows from the above definition of the skin treatment device according to the invention that, in comparison to what is the case in a conventional device such as the skin-heating unit known from WO 2022/069324 A1, the light source is mounted on another side of the carrier, namely on a side facing away from the skin-contacting surface, which is referred to as first side of the carrier, wherein light emitted by the light source during operation reaches the light-transmitting portion of the skin-contacting surface through reflection on an optical reflector. Further, the carrier is arranged such that at least a portion of the opposite side of the carrier, which is referred to as second side of the carrier, is mounted to an inner side of a wall portion of the housing including part of the skin-contacting surface, wherein said inner side is opposite to an outer side of said wall portion where said part of the skin-contacting surface is located, or the carrier is arranged such that said portion of the second side of the carrier constitutes part of the skin-contacting surface. A practical example of the carrier is a PCB. A practical example of the light source is a light emitting diode (LED). It may particularly be practical if the light source is configured to generate infrared or near-infrared light. Instead of infrared or near-infrared light, the treatment light might have a wavelength in the visible part of the light spectrum. For example, blue light may be generated to treat acne, and red light may be generated to stimulate wound healing. In such cases, the skin treatment device combines said light treatment with skin heating.

The fact that the treatment light reaches the light-transmitting portion of the skin-contacting surface through reflection by an optical reflector contributes to spreading of the light over the light-transmitting portion of the skin-contacting surface, so that it is possible to emit the treatment light more evenly to a larger part of the light-transmitting portion of the skin-contacting surface than in a situation in which the treatment light is emitted directly towards the light-transmitting portion of the skin-contacting surface. The invention covers any suitable type of optical reflector, and the optical reflector can be configured to reflect the treatment light towards the light-transmitting portion of the skin-contacting surface via a specular surface, a diffusive surface, or a combination of a specular surface and a diffusive surface. The fact that at least a portion of the second side of the carrier is mounted to an inner side of a wall portion of the housing including part of the skin-contacting surface or constitutes part of the skin-contacting surface contributes to an efficient conduction of the residual heat from the light source to the skin in contact with the skin-contacting surface. Advantageously, in the first case a distance between said portion of the second side of the carrier and said part of the skin-contacting surface included by said wall portion of the housing is equal to or only slightly larger than the thickness of said wall portion of the housing, while in the second case, said distance is even zero. For the sake of completeness, it is noted that in the second case, direct contact between said portion of the second side of the carrier and the skin under treatment is enabled. In any case, positioning the carrier in the skin treatment device in such a way that at least a portion of the second side of the carrier can be close to the skin-contacting surface or can even be part of the skin-contacting surface is facilitated by the fact that the treatment light is made to reach the light-transmitting portion of the skin-contacting surface indirectly, i.e., through reflection on an optical reflector. In the conventional case that the treatment light is made to reach the light-transmitting portion of the skin-contacting surface directly, the distance between the light source and the light-transmitting portion of the skin-contacting surface needs to be larger, and therefore the thermal path between the light source and the skin-contacting surface is considerably longer in the conventional case, which involves greater loss of the residual heat generated by the light source.

All in all, when the invention is applied, light and residual heat generated by the light source are conveyed to the skin under treatment in a most efficient manner. In this way, at least one of advantageous possibilities including a possibility to apply a relatively low-power light source, a possibility to apply a relatively light/small battery for powering the light source, and a possibility to make use of a charged battery for a relatively long time before the battery needs to be recharged is obtained.

In a practical embodiment of the skin treatment device according to the invention, the housing comprises an optically transparent wall including said light-transmitting portion of the skin-contacting surface. In the context of such an embodiment, one of the following two options can be chosen:
1) the second side of the carrier is mounted in direct contact with an inner surface of the optically transparent wall; or
2) the carrier is arranged in an opening provided in the optically transparent wall, and the skin-contacting surface comprises the second side of the carrier.
According to the first option, the wall portion of the housing, to which the second side of the carrier is mounted, is optically transparent.

It is further practical if a space in the skin treatment device which is present at a skin-facing side of the optical reflector and which accommodates the light source is filled with an optically transparent encapsulation material such as silicone hardened gel or another epoxy mixture. In order to ensure that reflected treatment light that falls on the carrier is optimally recycled and directed toward the light-transmitting portion of the skin-contacting surface, it is advantageous if the first side of the carrier is coated with a diffuse reflecting layer. Treatment of skin by means of light and heat from the light source of the skin treatment device can be optimized by designing the skin treatment device such that a thermal portion of the skin-contacting surface associated with the second side of the carrier is located adjacent to the light-transmitting portion of the skin-contacting surface, in other words, by designing the skin treatment device such that light and heat are emitted alongside each other from the skin-contacting surface during operation.

As mentioned earlier, it may particularly be practical if the light source is configured to generate infrared or near-infrared light. In this respect, a practical example of a wavelength range of the treatment light is a range of 900 nm to 1,100 nm. However, infrared or near-infrared light cannot be seen by humans. In such a case, in order to indicate an operational status of the skin treatment device to the user, use can be made of additional visible light. In this respect, the invention covers an embodiment of the skin treatment device which further comprises at least one indicator element configured to emit visible light via the light-transmitting portion of the skin-contacting surface when the light source emits the treatment light. The indicator element may have any suitable position in the skin treatment device, and may have a function of indicating an on/off status of the skin treatment device and possibly also a more sophisticated function such as a function of indicating intensity of the treatment light/heat. The indicator element may for example comprise an LED emitting visible light and mounted on the first side of the carrier adjacent to the at least one light source that generates the infrared or near-infrared treatment light.

A feasible application of the invention is an application in the field of electric shavers. In view thereof, the invention further relates to a shaving unit of an electric shaver, comprising a supporting member, at least one hair-cutting unit supported by the supporting member, and at least one skin treatment device as defined and described here before as skin treatment device according to the invention. It may particularly be so that the hair-cutting unit of the shaving unit is of the type comprising an external cutting member with hair-entry openings, an internal cutting member which is covered by the external cutting member and rotatable relative to the external cutting member, and a skin-supporting member surrounding the external cutting member, and that the skin treatment device is integrated into the skin-supporting member of the hair-cutting unit. Optimal use of available space in the skin-supporting member can be made if the skin treatment device comprises a plurality of light sources, and the light sources are positioned throughout the skin-supporting member in a configuration to at least partially surround the external cutting member. In case the shaving unit comprises at least two hair-cutting units, it is advantageous if the shaving unit comprises at least one skin treatment device associated with each hair-cutting unit, so that each of the hair-cutting units can be combined with at least one skin treatment device.

The invention also relates to an electric shaver comprising a main body and a shaving unit, coupled to the main body, as defined and described in the preceding paragraph.

The invention covers various ways in which operation of the skin treatment device may be controlled. For example, it is possible to have manual control of the skin treatment device, wherein the user is enabled to switch the skin treatment device on and off according to desire and/or to control light intensity. It is also possible to have automatic control of the skin treatment device. For example, in case the skin treatment device is used with a hair-cutting unit in a shaving unit, as suggested in the foregoing, it may be so that the skin treatment device is switched on and off along with the hair-cutting unit. The invention covers the use of any suitable user interface for enabling the user to control the on/off status of the skin treatment device and/or to set the light intensity and/or to select an automatic operation mode etc., which user interface may be present on an external device such as a smartphone.

The above-described and other aspects of the invention will be apparent from and elucidated with reference to the following detailed description of a skin treatment device as applied in a shaving unit of an electric shaver.

### BRIEF DESCRIPTION OF THE DRAWINGS

The invention will now be explained in greater detail with reference to the figures, in which equal or similar parts are indicated by the same reference signs, and in which:
Fig. 1 diagrammatically shows a perspective view of an electric shaver according to an embodiment of the invention, which electric shaver comprises a shaving unit and a main body;
Fig. 2 diagrammatically shows a perspective view of an external cutting member, a decorative cap and an internal cutting member of a hair-cutting unit of the shaving unit, wherein both the external cutting member and the cap are shown with a portion cut away;
Figs. 3 and 4 illustrate two options in respect of the set-up of a skin treatment device according to the invention, as integrated into a skin-supporting member of the hair-cutting unit and used on skin;
Figs. 5 and 6 diagrammatically show a perspective front view and a perspective back view, respectively, of an assembly of components of a shaving unit according to the invention, which shaving unit comprises a supporting member, three hair-cutting units supported by the supporting member, and skin treatment devices associated with each hair-cutting unit;
Fig. 7 diagrammatically shows a sectional view of a portion of the assembly of components of one of the hair-cutting units of the shaving unit and the skin treatment device associated with the hair-cutting unit;
Fig. 8 diagrammatically shows a perspective view of the assembly of components of the shaving unit, wherein the assembly is shown with a portion cut away;
Fig. 9 diagrammatically shows a perspective view of only the skin-supporting members and the skin treatment devices of the three hair-cutting units of the shaving unit; and
Figs. 10 and 11 diagrammatically show perspective views of only the skin-supporting member and the skin treatment device of one of the hair-cutting units of the shaving unit.

### DETAILED DESCRIPTION OF THE EMBODIMENTS

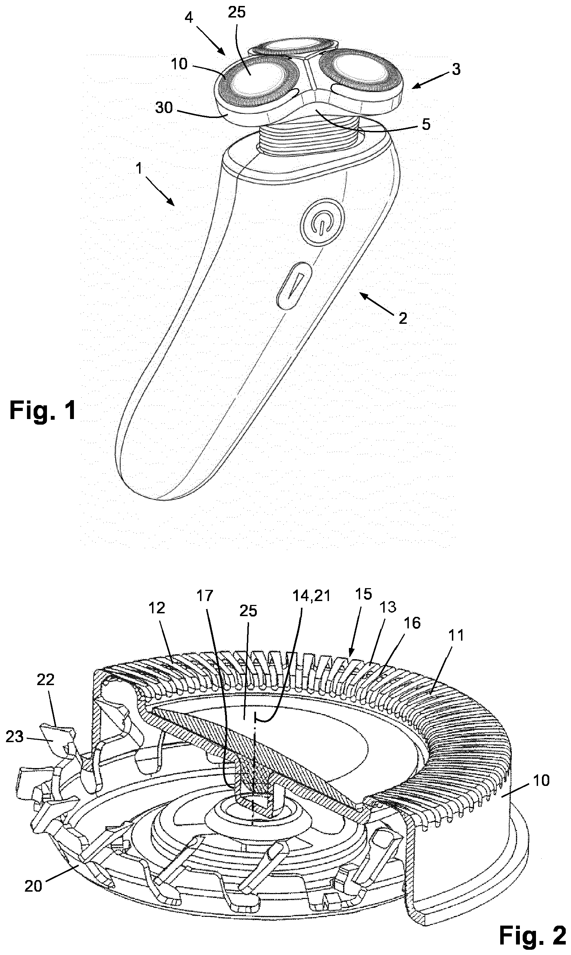

Fig. 1 shows an electric shaver of the rotary type, which is suitable to be used for shaving a beard, as a practical example of an electric shaver 1 according to an embodiment of the invention. The electric shaver 1 comprises a main body 2 and a shaving unit 3, wherein the main body 2 is designed to enable a user of the shaver 1 to take hold of the shaver 1 and to handle the shaver 1, and wherein the shaving unit 3 is the part of the shaver 1 that is to be positioned on and moved over the skin for hair removal. In the present example, the shaving unit 3 comprises three hair-cutting units 4 supported on a supporting member 5 of the shaving unit 3. When the electric shaver 1 is actually applied for the purpose of performing a shaving action, the actual process of cutting of hairs protruding from the skin takes place at the position of the hair-cutting units 4. In the context of the invention, the number of hair-cutting units 4 can be chosen freely and does not necessarily need to be three. The main body 2 may have a function in accommodating at least one electric motor for driving components of the respective hair-cutting units 4. The main body 2 may further include means such as a rechargeable battery for powering the at least one electric motor. It is practical if the shaving unit 3 and the main body 2 are releasably connectable to each other, as known per se in the field of electric shavers.

Each of the hair-cutting units 4 comprises a combination of an external cutting member 10 and an internal cutting member 20. Further, each of the hair-cutting units 4 comprises a skin-supporting member 30 which is arranged so as to surround the external cutting member 10.

Details of the combination of the external cutting member 10 and the internal cutting member 20 are now described with reference to Fig. 2. The external cutting member 10 is of a generally cup-shaped design and is thereby suitable for at least partially accommodating the internal cutting member 20 in its interior. The external cutting member 10 has an annular shaving track 11, an upper surface 12 of the shaving track 11 being intended to face and contact the skin to be subjected to a shaving action. The shaving track 11 comprises lamellae 13 extending along the width of the shaving track 11, in a substantially radial direction relative to a central axis 14 of the hair-cutting unit 4, which coincides with a rotational axis 21 about which the internal cutting member 20 is rotatable relative to the external cutting member 10. Apertures as present between the lamellae 13 constitute hair-entry openings 15 of the shaving track 11. Sides of the lamellae 13 constitute hair-cutting surfaces 16 suitable for cutting off hairs in cooperation with hair-cutting edges 22 of hair-cutting elements 23 of the internal cutting member 20. The invention also relates to cases in which the shaving track 11 does not comprise lamellae 13 or does not only comprise lamellae 13, such as cases in which the entire shaving track 11 is provided with teeth-like elements and/or a pattern of (circular) holes instead of or in addition to lamellae 13. Also, the invention relates to cases in which more than one shaving track 11 is present in the external cutting member 10.

A shaving action can be performed when the internal cutting member 20 is activated to rotate and a portion of skin is actually contacted by the external cutting member 10 at the position of the upper surface 12 of the shaving track 11. Activation of the internal cutting member 20 may take place in a known manner by means of a drive mechanism of the electric shaver 1. When the combination of the external cutting member 10 and the internal cutting member 20 is moved over the portion of skin while the internal cutting member 20 is driven to rotate, it is achieved that hairs protruding from the portion of skin are caught in the hair-entry openings 15 of the shaving track 11 of the external cutting member 10 and are cut off in that position as result of a cooperation between the hair-cutting surfaces 16 of the shaving track 11 of the external cutting member 10 and the hair-cutting edges 22 of the hair-cutting elements 23 of the rotating internal cutting member 20.

Besides the shaving track 11, the external cutting member 10 includes a central portion 17 comprising a central bearing portion which is designed to be used in rotationally supporting the internal cutting member 20 in the hair-cutting unit 4. The central portion 17 of the external cutting member 10 also serves for supporting a decorative cap 25 configured to cover part of the exterior surface of the external cutting member 10. In the present example, the central portion 17 comprises a centrally located recess, and the cap 25 comprises a projection which is accommodated in the recess.

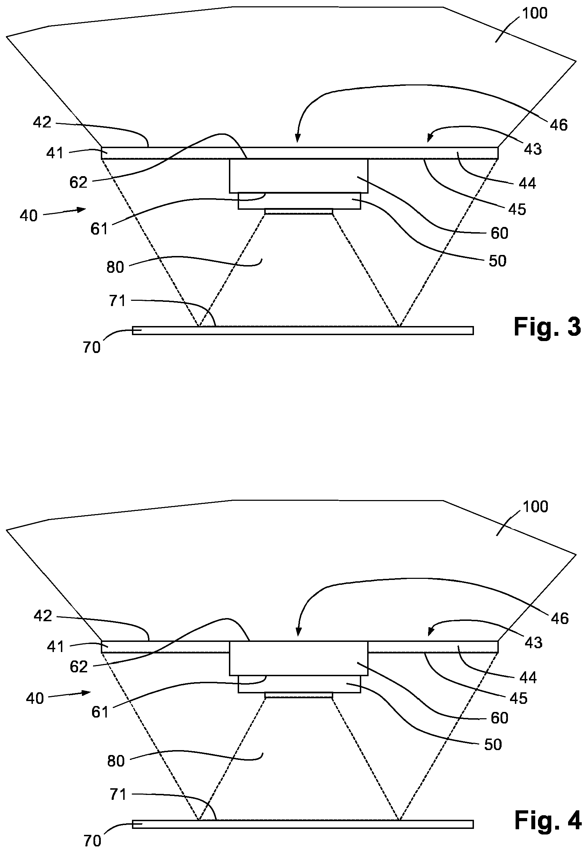

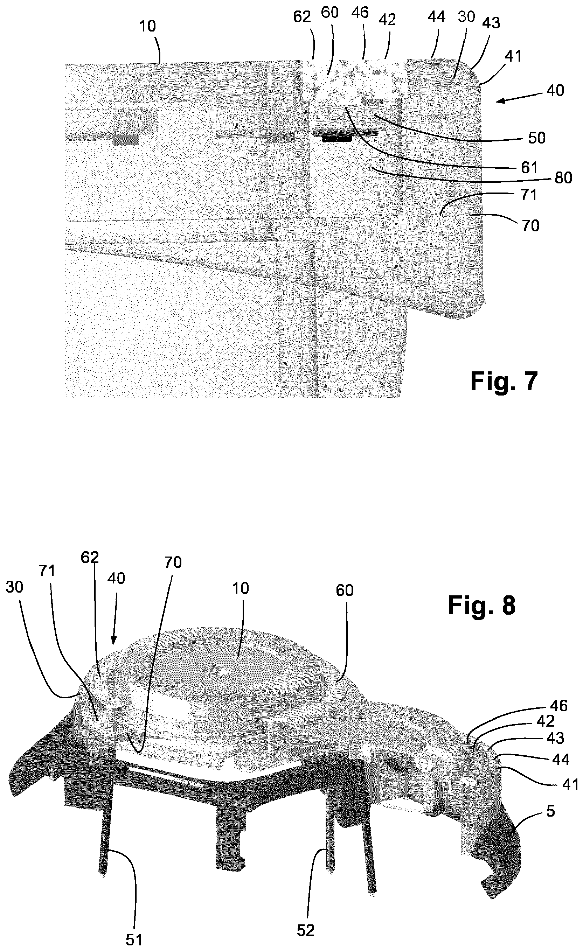

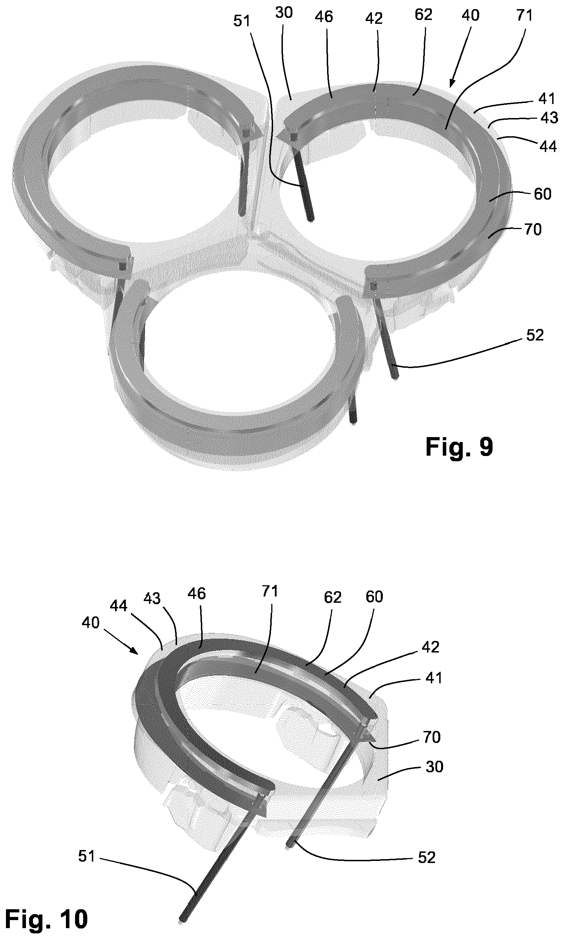

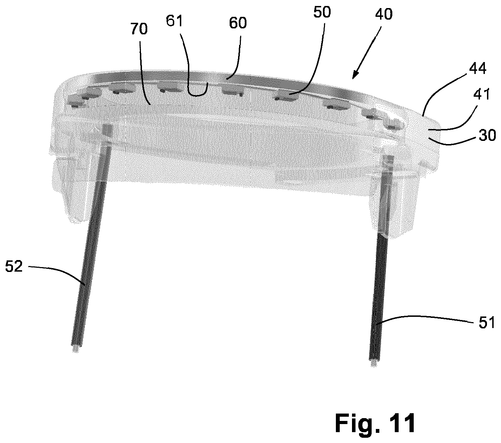

Each of the hair-cutting units 4 is equipped with a skin treatment device 40, as will now be explained in more details with reference to Figs. 3-11. Two options in respect of the set-up of the skin treatment device 40 are illustrated in Figs. 3 and 4. It is to be noted that the skin treatment device 40 does not necessarily need to be integrated in a structure such as a hair-cutting unit 4 of a shaving unit 3, although this is a practical option. Main components of the skin treatment device 40 are the following:
- a housing 41 provided with a skin-contacting surface 42 including a light-transmitting portion 43 which is optically transparent,
- at least one light source 50,
- a carrier 60 of the light source 50, and
- an optical reflector 70.

The skin-contacting surface 42 serves to contact skin 100 of the user, wherein it is noted that a portion of skin 100 is diagrammatically shown in Figs. 3 and 4, and that the functional positioning of the skin treatment device 40 with the skin-contacting surface 42 abutting against the skin 100 can be seen in the figures. During operation of the skin treatment device 40, the light source 50 is in an active state to generate treatment light to be applied to the skin 100 via the light-transmitting portion 43 of the skin-contacting surface 42. The light source 50 may be an LED, for example, particularly an LED which is configured to emit infrared light, near-infrared light, blue light or red light.

It is practical if the carrier 60 is a PCB. In any case, the light source 50 is mounted on a side 61 of the carrier 60 facing away from the skin-contacting surface 42, which side is referred to as first side 61 of the carrier 60. Thus, during operation of the skin treatment device 40, the treatment light is not directly emitted towards the light-transmitting portion 43 of the skin-contacting surface 42. Instead, the treatment light reaches the light-transmitting portion 43 of the skin-contacting surface 42 via the optical reflector 70, which is located at a distance from the skin-contacting surface 42, and which may comprise a specular surface, a diffusive surface, or a combination of a specular surface and a diffusive surface for reflecting the treatment light. In any case, the reflecting surface 71 of the optical reflector 70 may have any suitable shape, such as a planar shape, as shown in Figs. 3 and 4, or a curved shape. In Figs. 3 and 4, boundaries of the emitted light and the reflected light are indicated through dotted lines. Optionally, the carrier 60 is coated with a diffusive reflecting layer, so that treatment light falling on the carrier 60 can be optimally recycled and directed towards the skin 100.

In respect of the carrier 60, it is noted that the light source 50 is mounted on the first side 61 of the carrier 60, as explained in the foregoing, and that further at least a portion of the opposite side 62 of the carrier 60, which is referred to as second side 62 of the carrier 60, is in thermal contact with the skin-contacting surface 42 through a portion of the housing 41, as shown in Fig. 3, or constitutes part of the skin-contacting surface 42, as shown in Fig. 4. It is practical if at the skin interface, the housing 41 comprises an optically transparent wall 44, as shown in Figs. 3 and 4. Further, it is practical if the space between the optical reflector 70 and said optically transparent wall 44 is filled with an optically transparent encapsulation material 80. In Fig. 3, a first option of the second side 62 of the carrier 60 being mounted in direct contact with an inner surface 45 of said optically transparent wall 44 is illustrated, whereas in Fig. 4, a second option of the carrier 60 being arranged in an opening provided in said optically transparent wall 44 is illustrated. In respect of the second option, it is noted that if the carrier 60 is a PCB, indeed, the PCB may be of the type that is provided with a finish such as a metal finish at the second side 62, or with a suitable coating, so that an appealing overall look of the skin-contacting surface 42 can be realized even though the second side 62 of the carrier 60 is visible. In both options, two portions can be distinguished in the skin-contacting surface 42, namely the light-transmitting portion 43 and a thermal portion 46 at the position of the second side 62 of the carrier 60. In respect of the first option, it is noted that it is possible to apply a wall which is optically transparent except for an area that is at the position of the second side 62 of the carrier 60.

On the basis of the configuration of the skin treatment device 40 as described in the foregoing, it is achieved that a long optical path for the treatment light is created so that the treatment light may diverge more than in a situation in which the treatment light would be emitted directly towards the light-transmitting portion 43 of the skin-contacting surface 42, so that a larger area of the skin 100 is illuminated, wherein the light is spread over the light-transmitting portion 43 of the skin-contacting surface 42 so that hot spots are avoided. A further advantage of the configuration of the skin treatment device 40 as described in the foregoing resides in the fact that at least a portion and possibly all of the second side 62 of the carrier 60 is in close thermal contact with the skin-contacting surface 42 through the optically transparent wall 44 or constitutes part of the skin-contacting surface 42, as it is in relation to this fact that a thermal path from the light source 50 to the skin 100 can be as short as possible. Thus, on the basis of the configuration of the skin treatment device 40 as described in the foregoing, the output of the light source 50 in terms of generated light and heat is used in an effective and optimal fashion to realize treatment of the skin 100 by conductive thermal heating, and also by other phenomena such as deep optical-thermal heating in case the light source is configured to emit infrared or near-infrared light. Due to the short thermal path from the light source 50 to the skin 100, conductive heat transport from the light source 50 to the skin 100 is fast, and the user is enabled to experience the heating effect on the skin 100 right from the start of operation of the skin treatment device 40. Advantageously, the optically transparent wall 44 comprises a material having good thermal conductive properties besides being optically transparent or moderately scattering, such as sapphire or sintered/annealed alumina.

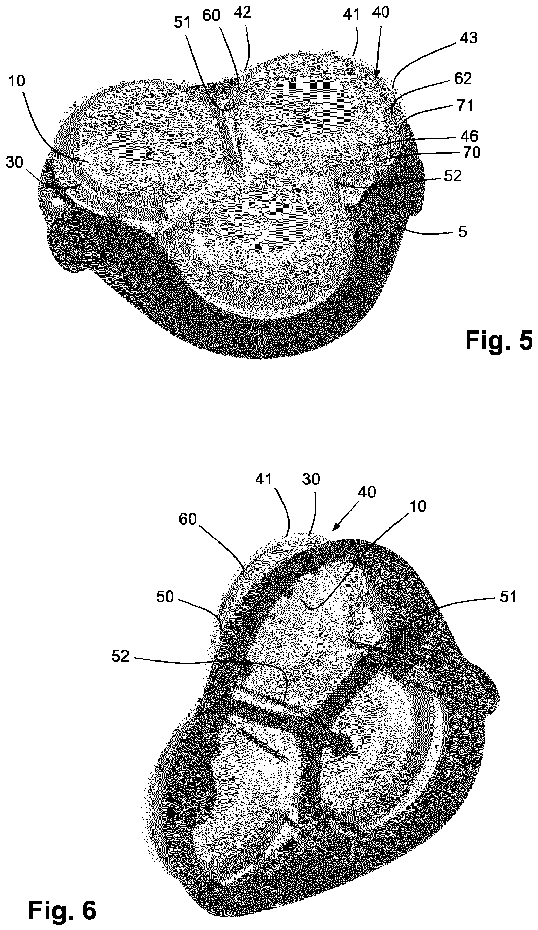

Aspects of the integration of skin treatment devices 40 in the hair-cutting units 4 of a shaving unit 3, especially in the skin-supporting member 30 of each of the hair-cutting units 4, become apparent from a consideration of Figs. 5-11. In the figures, two metallic pins 51, 52 of each of the skin treatment devices 40 for connection of the skin treatment device 40 to an energy source such as a battery of the electnc shaver 1 of which the shaving unit 3 is part can be seen. In the snown example, each of the treatment devices 40 comprises a plurality of light sources 50 mounted on a common carrier 60, wherein each of the light sources 50 is an LED and the carrier 60 is a PCB, and wherein the second option of the carrier 60 being arranged in an opening provided in the optically transparent wall 44 is applicable, so that the skin 100 is directly touched by the second side 62 of the carrier 60 when the shaving unit 3 is held against and/or moved over the skin 100. The optically transparent wall 44 is located at the outside of the skin-supporting member 30, and the optical reflector 70 is located in the interior of the skin-supporting member 30. In each of the hair-cutting units 4, the light sources 50 and the carrier 60 of the skin treatment device 40 are arranged so as to partially surround the external cutting member 10, so that the user can experience the skin treatment from areas of significant size around the areas where the shaving action takes place. Due to the geometry of the skin-supporting member 30, most of the treatment light is trapped inside the skin-supporting member 30 and can escape only in a situation of contact between the skin-contacting surface 42 and the skin 100, i.e. the very situation in which escape of the treatment light is envisaged for realizing the intended skin treatment.

It will be clear to a person skilled in the art that the scope of the invention is not limited to the examples discussed in the foregoing, and that several amendments and modifications thereof are possible without deviating from the scope of the invention as defined in the attached claims. It is intended that the invention be construed as including all such amendments and modifications insofar they come within the scope of the claims**.** While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in the present text will be understood by a person skilled in the art as covering the term "consist of". Hence, the term "comprise" or "include" may in respect of an embodiment mean "consist of", but may in another embodiment mean "contain/have/be equipped with at least the defined species and optionally one or more other species".

Notable aspects of the invention are summarized as follows. In the field of skin treatment, a device 40 is provided which is of the type comprising a housing 41 provided with a skin-contacting surface 42 arranged to be in contact with skin 100 of a user during use of the device 40. In the device 40, at least one light source 50 is arranged in the housing 41 for generating treatment light to be applied to the skin 100 via a light-transmitting portion 43 of the skin-contacting surface 42 which is optically transparent, wherein the light source 50 is mounted on a first side 61 of a carrier 60, said first side 61 facing away from the skin-contacting surface 42, and wherein the housing 41 accommodates an optical reflector 70 for reflecting the treatment light generated by the at least one light source 50 towards the light-transmitting portion 43 of the skin-contacting surface 42. Further, at least a portion of a second side 62 of the carrier 60 opposite to the first side 61 is mounted to an inner side of a wall portion of the housing 41 including part of the skin-contacting surface 42, or constitutes part of the skin-contacting surface 42, whereby a short thermal path from the light source 50 to the skin 100 is realized.

## Claims

1. Skin treatment device (40) comprising:
- a housing (41) provided with a skin-contacting surface (42) arranged to be in contact with skin (100) of a user during use of the device (40);
- at least one light source (50) arranged in the housing (41) for generating treatment light to be applied to the skin (100) via a light-transmitting portion (43) of the skin-contacting surface (42) which is optically transparent;
- a carrier (60) having a first side (61) on which the light source (50) is mounted, said first side (61) facing away from the skin-contacting surface (42);
- an optical reflector (70) accommodated by the housing (41) and arranged for reflecting the treatment light generated by the at least one light source (50) towards the light-transmitting portion (43) of the skin-contacting surface (42); wherein:
- at least a portion of a second side (62) of the carrier (60) opposite to the first side (61) of the carrier (60):
i) is mounted to an inner side of a wall portion of the housing (41), said wall portion including part of the skin-contacting surface (42), wherein said part of the skin-contacting surface (42) is located on an outer side of said wall portion which is opposite to said inner side of said wall portion; or
ii) constitutes part of the skin-contacting surface (42).

2. Skin treatment device (40) as claimed in claim 1, wherein the housing (41) comprises an optically transparent wall (44) including said light-transmitting portion (43) of the skin-contacting surface (42).

3. Skin treatment device (40) as claimed in claim 2, wherein the second side (62) of the carrier (60) is mounted in direct contact with an inner surface (45) of the optically transparent wall (44).

4. Skin treatment device (40) as claimed in claim 2, wherein:
- the carrier (60) is arranged in an opening provided in the optically transparent wall (44); and
- the skin-contacting surface (42) comprises the second side (62) of the carrier (60).

5. Skin treatment device (40) as claimed in any of claims 1-4, wherein a space in the skin treatment device (40) which is present at a skin-facing side of the optical reflector (70) and which accommodates the light source (50) is filled with an optically transparent encapsulation material (80).

6. Skin treatment device (40) as claimed in any of claims 1-5, wherein the first side (61) of the carrier (60) is coated with a diffuse reflecting layer.

7. Skin treatment device (40) as claimed in any of claims 1-6, wherein a thermal portion (46) of the skin-contacting surface (42) associated with the second side (62) of the carrier (60) is located adjacent to the light-transmitting portion (43) of the skin-contacting surface (42).

8. Skin treatment device (40) as claimed in any of claims 1-7, wherein:
- the light source (50) is an LED; and
- the carrier (60) is a printed circuit board (PCB).

9. Skin treatment device (40) as claimed in any of claims 1-8, wherein the light source (50) is configured to generate infrared or near-infrared light.

10. Skin treatment device (40) as claimed in claim 9, further comprising at least one indicator element configured to emit visible light via the light-transmitting portion (43) of the skin-contacting surface (42) when the light source (50) emits the treatment light.

11. Shaving unit (3) of an electric shaver, comprising a supporting member (5), at least one hair-cutting unit (4) supported by the supporting member (5), and at least one skin treatment device (40) as claimed in any of claims 1-10.

12. Shaving unit (3) as claimed in claim 11, wherein:
- the hair-cutting unit (4) comprises an external cutting member (10) with hair-entry openings (15), an internal cutting member (20) which is covered by the external cutting member (10) and rotatable relative to the external cutting member (10), and a skin-supporting member (30) surrounding the external cutting member (10); and
- the skin treatment device (40) is integrated into the skin-supporting member (30) of the hair-cutting unit (4).

13. Shaving unit (3) as claimed in claim 12, wherein:
- the skin treatment device (40) comprises a plurality of light sources (50); and
- the light sources (50) are positioned throughout the skin-supporting member (30) in a configuration to at least partially surround the external cutting member (10).

14. Shaving unit (3) as claimed in any of claims 11-13, comprising at least two hair-cutting units (4) and at least one skin treatment device (40) associated with each hair-cutting unit (4).

15. Electric shaver (1) comprising a main body (2) and a shaving unit (3) as claimed in any of claims 11-14, coupled to the main body (2).

## Patentansprüche

1. Hautbehandlungsvorrichtung (40), umfassend:
- ein Gehäuse (41), welches mit einer Hautkontaktfläche (42) versehen ist, welche so angeordnet ist, dass sie während der Verwendung der Vorrichtung (40) die Haut (100) eines Benutzers berührt;
- zumindest eine in dem Gehäuse (41) angeordnete Lichtquelle (50) zum Erzeugen von Behandlungslicht, welches über einen lichtdurchlässigen Abschnitt (43) der Hautkontaktfläche (42), welcher optisch transparent ist, auf die Haut (100) aufgebracht wird;
- einen Träger (60) mit einer ersten Seite (61), auf welcher die Lichtquelle (50) montiert ist, wobei die erste Seite (61) von der Hautkontaktfläche (42) abgewandt ist;
- einen optischen Reflektor (70), welcher in dem Gehäuse (41) untergebracht ist und dazu angeordnet ist, das von der zumindest einen Lichtquelle (50) erzeugte Behandlungslicht in Richtung des lichtdurchlässigen Abschnitts (43) der Hautkontaktfläche (42) zu reflektieren; wobei:
- zumindest ein Abschnitt einer zweiten Seite (62) des Trägers (60), welcher der ersten Seite (61) des Trägers (60) gegenüberliegt:
i) an einer Innenseite eines Wandabschnitts des Gehäuses (41) montiert ist, wobei der Wandabschnitt einen Teil der Hautkontaktfläche (42) beinhaltet, wobei sich der Teil der Hautkontaktfläche (42) auf einer Außenseite des Wandabschnitts befindet, welche der Innenseite des Wandabschnitts gegenüberliegt; oder
ii) einen Teil der Hautkontaktfläche (42) darstellt.

2. Hautbehandlungsvorrichtung (40) nach Anspruch 1, wobei das Gehäuse (41) eine optisch transparente Wand (44) umfasst, welche den lichtdurchlässigen Abschnitt (43) der Hautkontaktfläche (42) beinhaltet.

3. Hautbehandlungsvorrichtung (40) nach Anspruch 2, wobei die zweite Seite (62) des Trägers (60) in direktem Kontakt mit einer Innenfläche (45) der optisch transparenten Wand (44) montiert ist.

4. Hautbehandlungsvorrichtung (40) nach Anspruch 2, wobei:
- der Träger (60) in einer in der optisch transparenten Wand (44) bereitgestellten Öffnung angeordnet ist; und
- die Hautkontaktfläche (42) die zweite Seite (62) des Trägers (60) umfasst.

5. Hautbehandlungsvorrichtung (40) nach einem der Ansprüche 1-4, wobei ein Raum in der Hautbehandlungsvorrichtung (40), welcher sich auf einer der Haut zugewandten Seite des optischen Reflektors (70) befindet und die Lichtquelle (50) aufnimmt, mit einem optisch transparenten Kapselungsmaterial (80) gefüllt ist.

6. Hautbehandlungsvorrichtung (40) nach einem der Ansprüche 1-5, wobei die erste Seite (61) des Trägers (60) mit einer diffus reflektierenden Schicht beschichtet ist.

7. Hautbehandlungsvorrichtung (40) nach einem der Ansprüche 1-6, wobei sich ein thermischer Abschnitt (46) der Hautkontaktfläche (42), welcher der zweiten Seite (62) des Trägers (60) zugeordnet ist, neben dem lichtdurchlässigen Abschnitt (43) der Hautkontaktfläche (42) befindet.

8. Hautbehandlungsvorrichtung (40) nach einem der Ansprüche 1-7, wobei:
- die Lichtquelle (50) eine LED ist; und
- der Träger (60) eine Leiterplatte (PCB) ist.

9. Hautbehandlungsvorrichtung (40) nach einem der Ansprüche 1-8, wobei die Lichtquelle (50) dazu konfiguriert ist, Infrarot- oder Nahinfrarotlicht zu erzeugen.

10. Hautbehandlungsvorrichtung (40) nach Anspruch 9, weiter zumindest ein Anzeigeelement umfassend, welches dazu konfiguriert ist, sichtbares Licht über den lichtdurchlässigen Abschnitt (43) der Hautkontaktfläche (42) auszusenden, wenn die Lichtquelle (50) das Behandlungslicht aussendet.

11. Rasiereinheit (3) eines Elektrorasierers, umfassend ein Auflageelement (5), zumindest eine Haarschneideeinheit (4), welche von dem Auflageelement (5) getragen wird, und zumindest eine Hautbehandlungsvorrichtung (40) nach einem der Ansprüche 1-10.

12. Rasiereinheit (3) nach Anspruch 11, wobei:
- die Haarschneideeinheit (4) ein äußeres Schneideelement (10) mit Haardurchgangsöffnungen (15), ein inneres Schneideelement (20), welches von dem äußeren Schneideelement (10) bedeckt ist und relativ zu dem äußeren Schneideelement (10) drehbar ist, und ein Hautauflageelement (30) umfasst, welches das äußere Schneideelement (10) umgibt; und
- die Hautbehandlungsvorrichtung (40) in das Hautauflageelement (30) der Haarschneideeinheit (4) integriert ist.

13. Rasiereinheit (3) nach Anspruch 12, wobei:
- die Hautbehandlungsvorrichtung (40) eine Vielzahl von Lichtquellen (50) umfasst; und
- die Lichtquellen (50) in dem gesamten Hautauflageelement (30) in einer Konfiguration positioniert sind, um das äußere Schneideelement (10) zumindest teilweise zu umgeben.

14. Rasiereinheit (3) nach einem der Ansprüche 11-13, umfassend zumindest zwei Haarschneideeinheiten (4) und zumindest eine jeder Haarschneideeinheit (4) zugeordnete Hautbehandlungsvorrichtung (40).

15. Elektrorasierer (1), umfassend einen Hauptkörper (2) und eine Rasiereinheit (3) nach einem der Ansprüche 11-14, welche an den Hauptkörper (2) gekoppelt ist.

## Revendications

1. Dispositif de traitement de la peau (40) comprenant :
- un boîtier (41) pourvu d'une surface de contact avec la peau (42) agencé pour être en contact avec la peau (100) d'un utilisateur pendant l'utilisation du dispositif (40) ;
- au moins une source de lumière (50) agencée dans le boîtier (41) pour générer une lumière de traitement à appliquer sur la peau (100) via une partie de transmission de lumière (43) de la surface de contact avec la peau (42) qui est optiquement transparente ;
- un support (60) présentant un premier côté (61) sur lequel la source de lumière (50) est montée, ledit premier côté (61) étant orienté à l'écart de la surface de contact avec la peau (42) ;
- un réflecteur optique (70) logé dans le boîtier (41) et agencé pour réfléchir la lumière de traitement générée par la au moins une source de lumière (50) vers la partie de transmission de lumière (43) de la surface de contact avec la peau (42) ; dans lequel :
- au moins une partie d'un second côté (62) du support (60) opposé au premier côté (61) du support (60) :
i) est montée sur un côté interne d'une partie de paroi du boîtier (41), ladite partie de paroi incluant une partie de la surface de contact avec la peau (42), dans lequel ladite partie de la surface de contact avec la peau (42) est située sur un côté externe de ladite partie de paroi qui est opposé audit côté interne de ladite partie de paroi ; ou
ii) constitue une partie de la surface de contact avec la peau (42).

2. Dispositif de traitement de la peau (40) selon la revendication 1, dans lequel le boîtier (41) comprend une paroi optiquement transparente (44) incluant ladite partie de transmission de lumière (43) de la surface de contact avec la peau (42).

3. Dispositif de traitement de la peau (40) selon la revendication 2, dans lequel le second côté (62) du support (60) est monté en contact direct avec une surface interne (45) de la paroi optiquement transparente (44).

4. Dispositif de traitement de la peau (40) selon la revendication 2, dans lequel :
- le support (60) est agencé dans une ouverture ménagée dans la paroi optiquement transparente (44) ; et
- la surface de contact avec la peau (42) comprend le second côté (62) du support (60).

5. Dispositif de traitement de la peau (40) selon l'une quelconque des revendications 1-4, dans lequel un espace dans le dispositif de traitement de la peau (40) qui est présent au niveau d'un côté faisant face à la peau du réflecteur optique (70) et qui loge la source de lumière (50) est rempli d'un matériau d'encapsulation optiquement transparent (80).

6. Dispositif de traitement de la peau (40) selon l'une quelconque des revendications 1-5, dans lequel le premier côté (61) du support (60) est recouvert d'une couche de réflexion diffuse.

7. Dispositif de traitement de la peau (40) selon l'une quelconque des revendications 1-6, dans lequel une partie thermique (46) de la surface de contact avec la peau (42) associée au second côté (62) du support (60) est située adjacente à la partie de transmission de lumière (43) de la surface de contact avec la peau (42).

8. Dispositif de traitement de la peau (40) selon l'une quelconque des revendications 1-7, dans lequel :
- la source de lumière (50) est une LED ; et
- le support (60) est une carte de circuit imprimé (PCB).

9. Dispositif de traitement de la peau (40) selon l'une quelconque des revendications 1-8, dans lequel la source de lumière (50) est configurée pour générer une lumière infrarouge ou proche infrarouge.

10. Dispositif de traitement de la peau (40) selon la revendication 9, comprenant en outre au moins un élément indicateur configuré pour émettre une lumière visible via la partie de transmission de lumière (43) de la surface de contact avec la peau (42) lorsque la source de lumière (50) émet la lumière de traitement.

11. Unité de rasage (3) d'un rasoir électrique, comprenant un élément de support (5), au moins une unité de coupe de poils (4) supportée par l'élément de support (5) et au moins un dispositif de traitement de la peau (40) selon l'une quelconque des revendications 1-10.

12. Unité de rasage (3) selon la revendication 11, dans laquelle :
- l'unité de coupe de poils (4) comprend un élément de coupe externe (10) avec des ouvertures d'entrée de poils (15), un élément de coupe interne (20) qui est recouvert par l'élément de coupe externe (10) et qui peut tourner par rapport à l'élément de coupe externe (10) et un élément de support de peau (30) entourant l'élément de coupe externe (10) ; et
- le dispositif de traitement de la peau (40) est intégré dans l'élément de support de peau (30) de l'unité de coupe de poils (4).

13. Unité de rasage (3) selon la revendication 12, dans laquelle :
- le dispositif de traitement de la peau (40) comprend une pluralité de sources de lumière (50) ; et
- les sources de lumière (50) sont positionnées dans tout l'élément de support de peau (30) dans une configuration pour entourer au moins partiellement l'élément de coupe externe (10).

14. Unité de rasage (3) selon l'une quelconque des revendications 11-13, comprenant au moins deux unités de coupe de poils (4) et au moins un dispositif de traitement de la peau (40) associé à chaque unité de coupe de poils (4).

15. Rasoir électrique (1) comprenant un corps principal (2) et une unité de rasage (3) selon l'une quelconque des revendications 11-14, couplée au corps principal (2).
